# EUROPEAN PATENT SPECIFICATION

(11) **EP 3 143 937 B1**
(45) Date of publication and mention of the grant of the patent: **25.03.2020**
(21) Application number: 16187404.5
(22) Date of filing: 06.09.2016
(51) Int. Cl.: A61B 6/06, A61B 6/00, A61B 6/02, A61B 6/04, A61B 6/12

(54) **SYSTEM TO IMAGE BIOPSY SAMPLES DURING AN X-RAY GUIDED BIOPSY PROCEDURE**
SYSTEM FÜR BILDBIOPSIEPROBEN WÄHREND EINER RÖNTGENGEFÜHRTEN BIOPSIEPROZEDUR
SYSTEME POUR ECHANTILLONS DE BIOPSIE EN IMAGE PENDANT LA PROCEDURE DE BIOPSIE GUIDEE PAR RAYONS X

(30) Priority: 21.09.2015 US 201514860303
(43) Date of publication of application: 22.03.2017
(73) Proprietor: General Electric Company, Schenectady, NY 12345 (US)
(72) Inventor: IORDACHE, Razvan Gabriel, 78530 FR Buc (FR); VANCAMBERG, Laurence, 78530 FR Buc (FR)
(74) Representative: Fennell, Gareth Charles

(56) References cited:
- EP-A1- 2 277 445
- EP-A1- 2 772 191
- US-A1- 2009 225 935

## Description

The development of imaging parts of the human body has been instrumental in modern diagnostic and surgical procedures within the medical field. This imaging allows medical professionals to detect and monitor abnormalities and suspicious masses in an accurate manner that is less invasive than surgery. In addition to accurate diagnostics, medical centers and medical professionals must also consider the efficiency of the procedure and the cost of the imaging devices when choosing what imaging equipment to utilize.

The techniques discussed herein can be applied to various x-ray imaging systems, for example, to mammography and breast biopsy systems, such as those described in EP 2 277 445 and US 2009/0225935. In a typical mammography procedure, x-ray images are generated of a patient's breast in order to identify suspicious masses or abnormalities. If a medical professional confirms the presence of a suspicious mass, further imaging may be performed to obtain additional information about the mass. In some cases, a breast biopsy is performed, particularly if it is believed the mass is malignant.

In a breast biopsy, a needle is typically inserted into the breast manually or by using automatic means, such as during a stereotactic x-ray biopsy. In a typical biopsy procedure, a patient can be positioned on a breast support located above an x-ray detector and below an x-ray source. The breast is then held in place by a compression paddle for more accurate imaging of the breast. A biopsy instrument may be used manually and/or with an imaging system that proposes coordinates of a region of interest to acquire biopsy tissue samples of a region of interest.

To determine whether the samples acquired contain malignant or suspicious masses, a user may use an imaging system to acquire images of the samples themselves. Currently, a user must acquire the samples then bring the samples to a separate imaging machine. The separate imaging machine may be located in a room dedicated to sample imaging analysis or the machine may be another imaging machine that is not currently in use on another patient. Alternatively, some imaging systems contain separate, dedicated imaging means including a separate x-ray detector and x-ray source for imaging the biopsy samples only. These separate imaging means are typically attached to the imaging system and are dedicated to a certain field within which a user must place the acquired samples.

A problem of the method and systems described above is that additional x-ray imaging devices are required, which (i) may not be located in the same room as the ongoing biopsy procedure, (ii) may be in use on another patient, and/or (iii) may require a separate, dedicated sample imaging apparatus. If the additional imaging devices are being used to perform other procedures, a patient, who is already undergoing a long and uncomfortable procedure, may be forced to wait in an uncomfortable position for an extended period of time. As an alternative, dedicated sample imaging means may be used to avoid this uncomfort and/or the transport of samples to a different machine or different location; however, the dedicated sample imaging means increase the cost of the machine. Furthermore, dedicated sample imaging means may break or require maintenance at both a financial cost and downtime cost to a medical facility.

In accordance with this disclosure, a system for imaging biopsy samples during an x-ray guided biopsy procedure as defined by the appended claims is provided. More particularly, this disclosure relates to improved systems for imaging breast biopsy samples during an x-ray guided breast biopsy procedure. The systems described herein allow for the imaging of biopsy samples during the biopsy procedure on the same imaging machine where the patient is imaged without the need to move the patient from the imaging machine.

The x-ray system and disclosed herein does not require the transfer of the samples being imaged to a different imaging system and does not require dedicated sample imaging means. Instead, the x-ray system provides a user with the technical advantage of imaging both the object and the biopsy samples from the object using the same imaging system without moving the patient. This allows for a patient, for example a woman undergoing a breast biopsy, to remain in position and under compression while biopsy samples are taken, while the biopsy samples are imaged, and while additional samples are taken or a full biopsy is performed. The x-ray system, therefore, reduces financial costs for additional equipment, downtime, procedure time, and possible sample mix-ups where multiple machines are used.

The various embodiments of this x-ray system, described in more detail below, provide a number of options for the user of the imaging system to image the samples. Advantageously, the embodiments limit the amount of radiation a patient or object is exposed to while making the procedure more efficient. Furthermore, the embodiments allow for automatic, system, and manual adjustment of the field of view for imaging the object or patient, as well as the samples. This flexibility allows a user to ensure the field of view of the samples is large enough to accommodate the samples and to adjust the collimator to exclude as much of the object or patient as possible, at least a portion of the object, or a majority of the object or patient.

By providing a light source that corresponds to the field of view of the collimator as discussed herein, the user can visualize which portion of the object support will be exposed to the x-rays. A user can then manually adjust the field of view using mechanisms located on the collimator or adjust the light source, which corresponds to the field of view. This will allow the user to visualize where the samples should be placed once the collimator is adjusted or allow the user to adjust the field of view to fit the samples. After the adjustment, the same system used to acquire the image of the object or patient, for example a scout image of the object is used to acquire at least one image of the samples without the need for additional imaging equipment.

Commercial advantages provided by the x-ray system discussed herein include providing a simpler, easier, and lower cost means of imaging a biopsy samples in contrast to the existing techniques. Currently, additional x-ray imaging devices are required to image biopsy samples, either by performing the imaging on a different machine altogether, or by using a separate, dedicated biopsy sample imaging system. This additional equipment is costly, requires upkeep and maintenance, and can add time to the biopsy procedure.

Various aspects and embodiments of the present invention are thus defined by the appended claims.

The operation of the inventive methods and systems will become apparent from the following description taken in conjunction with the drawings, in which:
Figure 1 illustrates an exemplary x-ray system;
Figure 2 illustrates a top view of an object being imaged on an object support;
Figure 3 illustrates an interactive interface on a display;
Figure 4 illustrates a top view of an object being imaged on an object support, which is divided into sections;
Figure 5 illustrates a top view of an object being imaged on an object support, which is divided into sections and includes samples to be imaged;
Figure 6 illustrates an interactive interface on a display;
Figure 7 illustrates a top view of an object being imaged on an object support and a proposed field of view;
Figure 8 illustrates a top view of an object being imaged on an object support and a proposed field of view in which samples have been placed;
Figure 9 illustrates a field of view of the x-ray source;
Figure 10 illustrates a field of view of the x-ray source and illumination means;
Figure 11 illustrates a field of view of the x-ray source and illumination means;
Figure 12 illustrates a top view of an object being imaged and multiple biopsy sample imaging areas;
Figure 13 illustrates a field of view of the x-ray source and illumination means;
Figure 14 illustrates a flow chart of an exemplary method of imaging biopsy samples using a user interface; and
Figure 15 illustrates a flow chart of an exemplary method of imaging biopsy samples.

The invention discussed herein allows for the imaging of tissue biopsy samples during a biopsy procedure on the same machine where an object or patient is imaged, without the need to move or remove the patient or the portion of the patient being imaged, such as a patient's breast.

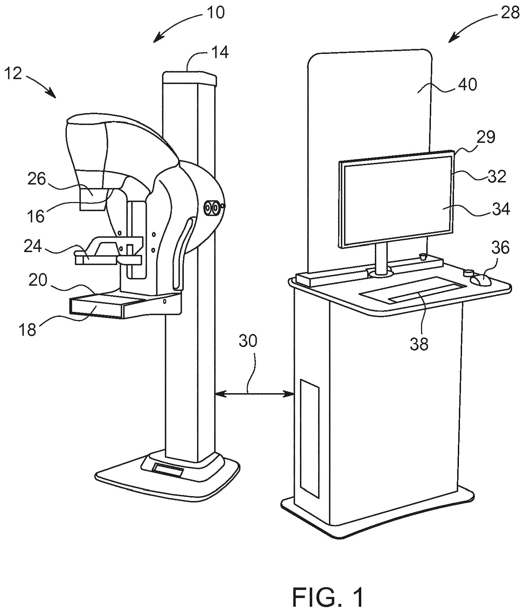

Referring to Figure 1, an image of an exemplary x-ray system is shown. The x-ray system 10 includes an x-ray apparatus 12. The x-ray apparatus 12 may be a tomosynthesis apparatus, such as the digital breast tomosynthesis ("DBT") apparatus shown in Figure 1. Though reference is made herein to breast biopsies and DBT, it is to be understood that the systems and methods described herein may be applied to other similar uses in the field of x-ray imaging and tomography, and is not limited to the field of DBT or breast biopsies.

The x-ray apparatus 12 includes a support structure 14, to which an x-ray source 16 and an x-ray detector 18 may be attached. The x-ray source 16 is directed toward a volume or object 22 to be x-rayed, and is configured to emit a beam of x-rays at desired times and to acquire one or more x-ray images 42. Once the x-rays are emitted, the beam of x-rays pass through the object 22 and are picked up by or hit the detector 18. The detector 18 may be any one of a variety of different detectors conventionally known in the art or that will become available in the future, such as an x-ray detector, digital radiography detector, or flat panel detector.

The x-ray apparatus 12 may also include an object support 20 on which the object 22 is placed. The object 22 may be a portion of a patient, such as a breast. The x-ray apparatus 12 may further include compression means, such as a compression paddle 24, which compresses the object 22. Compression paddle 24 holds the breast still and in place while optionally providing apertures to allow for insertion of a biopsy tool, needle, core needle, or vacuum assisted core needle. Compression paddle 24 also serves to compress the object or breast to minimize the thickness traversed by the x-rays and to help reduce movement of the object 22 due to the patient moving. Optionally, the x-ray apparatus 12 may also include a patient shield 26 to shield portions of a patient from radiation exposure from the x-ray source and/or to shield the patient from viewing the procedure and biopsy tissue samples.

In addition to the x-ray apparatus 12, the x-ray system 10 may include a work station 28, which includes a computer or controller 29. The work station 28 and/or controller 29 is connected or in communication with at least the x-ray apparatus 12, through connection 30, which could be wired or wireless. The controller 29 may also be connected to a display 32 comprising an interface 34, which allows a user to interact with the controller 29 to acquire x-ray images, position and operate parts of the x-ray apparatus 12, define or propose a region of interest or field of view, and display images 42. The controller 29, which may be a processor, computer, etc., may include a memory or storage and processing circuitry that executes stored program logic and may be any one of a different computers, processors, controllers, or combination thereof that are available for and compatible with the various types of equipment and devices used in the x-ray system 10.

Through its processors and controllers, the controller 29 controls the operation and function of the x-ray source 16 and the detector 18. The controller 29 is capable of controlling when the x-ray source 16 emits x-rays, how the detector 18 reads and conveys information or signals after the x-rays hit the detector 18, and how the x-ray source 16 and the detector 18 moves relative to one another and relative to the object 22. The controller 29 also controls how information, including images 42 and data acquired during the operation, is processed, displayed, stored, and manipulated. The different processing steps performed by the controller 29 are dictated and controlled by software designed to allow controller 29 to perform the various operations underlying the acquisition of x-rays, some of which are discussed herein. Information may also be stored in one or more memories of controller 29 for later retrieval and use.

The work station 28 typically includes one or more of the display 32 with the interface 34, selecting means 36, input means 38, and a radiation shield 40. The work station 28 can be used by an x-ray technician, physician, or other trained professional for interacting with the x-ray apparatus 12 as a user. The input means 38 may be a mouse, keyboard, touch pad, or other input devices. The display 32 can display one or more images 42 at the same time, different times, in sequence, or out of sequence that were acquired during an x-ray procedure, or track data relevant to the x-ray system 10 from controller 29. The display coupled to the workstation 28 may be utilized to observe the images 42 and to control scout image acquisition, further image acquisition, which may be used to guide a biopsy procedure, and/or imaging of biopsy samples as discussed herein.

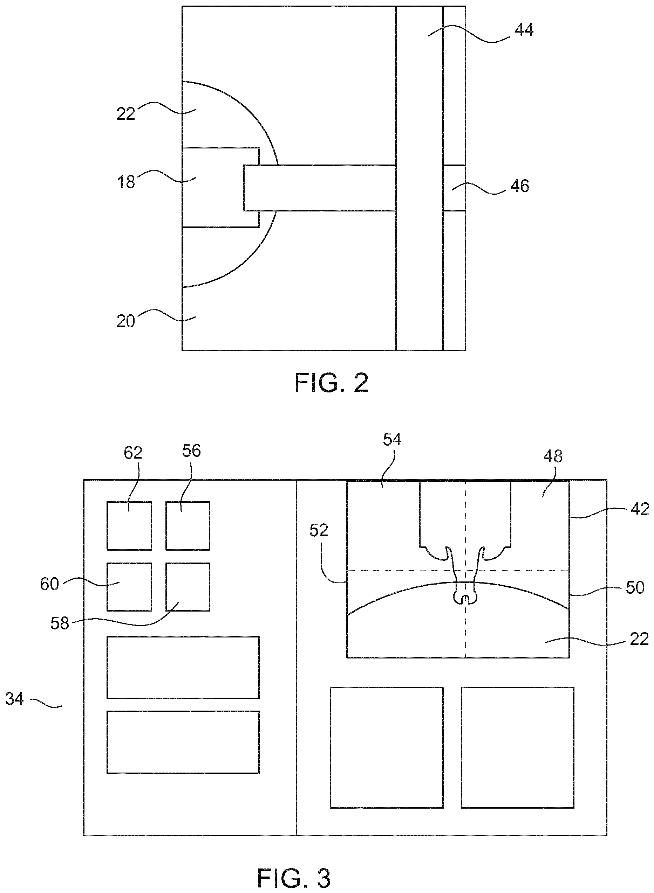

With reference to Figures 2, 4, 5, 7, 8, and 12, an image of a top view of the x-ray apparatus 12 is shown. A structure 44 is depicted, which is a part of the support 14 shown in Figure 1. The structure 44 may house one or more of an x-ray source, a collimator, illumination means, and/or other elements used for image acquisition (not shown). Also shown in Figures 2, 4, 5, 7, 8, and 12 is the object 22, the detector 18, the object support 20, and a biopsy positioning device 46. The biopsy positioning device aids in the positioning of a biopsy tool or biopsy needle.

In an embodiment, the biopsy tool may be configured as a biopsy needle mounted to a hand piece. The biopsy needle may include an outer cannula, an inner cannula positioned therein, and an opening for receiving a portion of tissue from the biopsied lesion or target. The cannulas form a cutting device wherein the outer cannula is configured to slide or rotate over the inner cannula, and/or the inner cannula is configured to slide or rotate within the outer cannula. The biopsy tool may further comprise an introducer stylet and an introducer sheath mounted to the hand piece. The introducer stylet inserted within the introducer sheath is inserted into a patient.

Once the biopsy tool is inserted, at least one biopsy sample may be removed from object 22, such as a patient's breast, by use of aspiration and/or the cutting mechanism formed by the inner and outer cannulas. The sample is moved by aspiration down an aspiration tube coupled to a collection chamber with individual prelabeled chambers to delineate the order or location of each sample from the biopsy procedure. Alternative means of labeling each sample allowing for location identification and/or order identification may also be employed.

Figure 3 illustrates the interface 34 on which one or more images 42, is displayed. The one or more images 42 may be a scout image acquired prior to a biopsy, may be an image acquired during the biopsy to guide the user of the system, and/or may be more than one image acquired before and/or during the biopsy procedure. One or more images 42 in Figure 3 corresponds to the top view of the object 22 positioned on an object support 20 as shown in Figure 2. In a typical biopsy procedure, a scout image is acquired, the biopsy positioning device is adjusted using the scout image so that a biopsy tool is positioned to reach the target, such as a lesion or tumor, and biopsy samples are acquired.

In an embodiment, the x-ray system 10, particularly controller 29, proposes a region of interest corresponding to the field of view from at least one predefined region, for acquiring images of biopsy samples positioned within the predefined region. As shown in Figure 3, the system 10 may divide the one or more images 42 into four distinct quadrants, region one 48, region two 50, region three 52, and region four 54. The x-ray system is not limited to quadrants and may divide a field of view into two, three, four, six, eight, or more predefined regions or sections corresponding to a region of interest and field of view. The predefined regions may be all of equal size and shape, or may have at least one region differing in size or shape from the other regions.

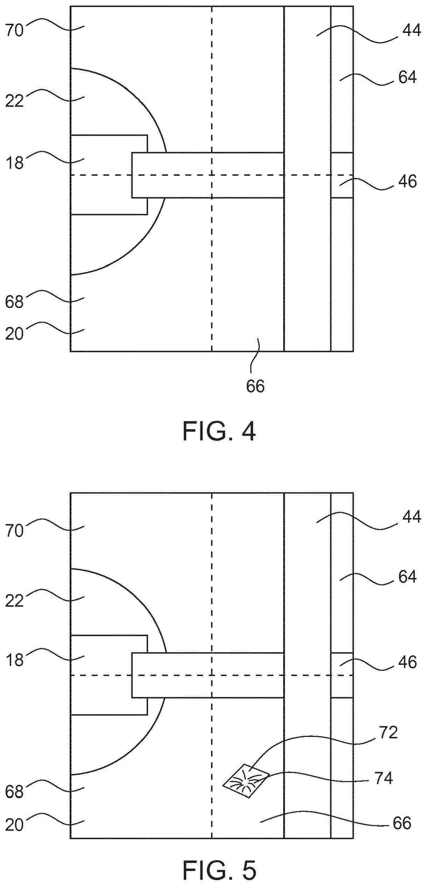

As illustrated by dotted lines in Figure 4, the field of view of the x-ray source 16 is divided into regions corresponding to the regions of interest of Figure 3, for example quadrants, in which biopsy samples can be placed for imaging. Regions 64, 66, 68, and 70 of the field of view in Figure 4 correspond to the regions 48, 50, 52, and 54 depicted in Figure 3. The x-ray apparatus 12 will acquire images of only the selected region(s), for example one region 66, omitting all other non-selected regions (Figure 3) from the field of view.

In one embodiment, the x-ray system 10 may propose one of the four regions, 48, 50, 52, and 54 from the predefined regions on the interface 34 as a default position. This embodiment may save time and is advantageous when a user typically places the biopsy samples in a particular region for many patients based on known positioning of the patient on the x-ray apparatus 12. As illustrated in Figure 3, regions 48 and 54 would be more likely to be selected as default regions than regions 50 and 52, which contain the object 22. The x-ray system may automatically select this default position or may ask a user to confirm whether or not they would like to proceed with the default position.

In another embodiment, the system may propose one of the regions based on knowledge of the current position of the object 22 on the object support 20 and/or by image analysis. In this embodiment, an automatic object detection algorithm may be applied to identify the area of the image covered by the object 22. The object 22 may be a breast and the image can be a 2D projection image of the breast. In an example of this embodiment, the pixels belonging to the breast may be separated from the pixels belonging to the background by "thresholding." In thresholding, the computer computes the coordinates of at least one region in the background which can be used for biopsy sample imaging.

The system may also use a method for extrapolating a grey-level bidimensional image, such as that disclosed in U.S. Patent No. 8,098,920 B2 to General Electric Company. In an example of this embodiment, a field of the bidimensional grey-level image to be extrapolated is determined where at each point on the field a grey-level value is known and pertinent. Then, a bidimensional mask associated with the field and extrapolated beyond the limit of a recording means is determined. The bidimensional grey-level image beyond the limit of the recording means from the extrapolated bidimensional mask is then extrapolated. It should be understood that the image analysis and knowledge of the current position of the object 22 on the detector 24 is not limited to thresholding and/or the example provided relating to U.S. Patent No. 8,098,920 B2, and that a person having ordinary skill in the art may employ additional or different methods.

In addition or alternatively to the system 10 proposing a region automatically to a user, the system 10, specifically the interface 34, may also include one or more, or a plurality of tool icons 47 for interacting with the one or more of the one or more images 42 and/or any portion of the x-ray system 10, such as the x-ray source 16. Tool icons discussed herein are non-limiting examples as to their position, shape, size, and location on the interface 34. It should be understood that additional or fewer tool icons may be shown and that tool icons may be used that accomplish the same result as discussed herein but are called a different name.

A user may use one or more of the tool icons 47 to select a quadrant or predefined region. As an example, tool icons 47 may comprise specific region selection tools 56, 58, 60, and 62, which correspond to the regions 48, 50, 52, and 54 of the field of view of the one or more images 42. For example, a user may select region 48 using quadrant selection tool 56 or may select regions 48 and 54 together using quadrant selection tools 56 and 62 together to select a larger field of view in which to place biopsy samples. Alternatively, a user may use region selection tools 56, 58, 60, or 62 to move from a proposed default region to a different region after the user has analyzed the one or more images 42, effectively overriding the default region.

In an exemplary embodiment, a user may select region 48 (shown in Figure 3 for example) using the region selection tool 56, which corresponds to the field of view region 66 (shown in Figures 4 and 5, for example). As shown in Figure 5, a user may then place one or more biopsy samples 74 into field of view region 66 for imaging. The one or more biopsy samples 74 may be placed directly onto the object support 20 or may be positioned onto a sample holder 72. The sample holder 72 may be comprised of any material sufficient to hold the biopsy samples 74. For example, a cloth may be used, a plastic container, or other means which allow for x-rays to pass from the x-ray source 16 to the x-ray detector 18 and does not substantially interfere with the image acquisition of the x-ray system 10.

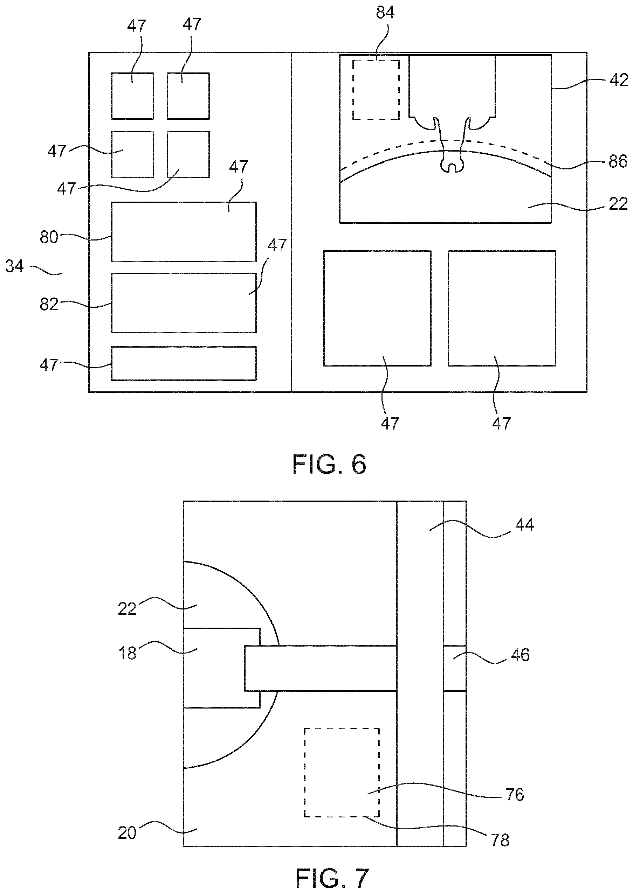

Referring to Figure 6, another embodiment of the interface 34 of x-ray system 10 is shown. In this embodiment, rather than the x-ray system 10 proposing a region in which to image biopsy samples or a user selecting a predefined region, the user selects or draws a region of interest in which to image biopsy samples. Interface 34 in this embodiment contains tool icons 47, and specifically at least one of a region of interest tool 80 and/or an object tool 82.

The region of interest tool 80 allows a user to look at the one or more images 42 of object 22 and draw a region of interest 84 corresponding to a field of view of the x-ray source 16 to (i) keep the object outside of the field of view altogether or as much as possible and/or (ii) draw or select a region of interest large enough to accommodate the samples. A user may select the region of interest tool 80 to draw a region of interest 84 on one or more images 42. In one embodiment, the region of interest tool 80 allows a user to draw a field of view of any shape or size within the boundaries of the one or more images 42. In another embodiment, the region of interest tool 80 allows a user to draw a region of interest 84, such as a rectangle, circle, square, polygon, or other shape, and to adjust the size and position of this shape. In order to draw the shape, a user may select, draw, and/or adjust the drawn region of interest 84 using a mouse, keyboard, touch pad, touch screen, or other means for drawing and selecting.

As shown in Figure 6, a rectangular region of interest 84 is drawn on the one or more images 42 which also includes the object 22 and the biopsy positioning device 46. The rectangular field of view shape 84 corresponds with the field of view of the x-ray source 16 on the x-ray apparatus 12, which limits the radiation exposure to the field of view.

With reference to Figure 7, the top view of the object 22 being imaged is shown with the limited field of view defined using the interface 34 shown in Figure 6. As shown, a sample region of interest 76 comprises a sample region of interest boundary 78. The field of view of the x-ray source 16 is limited to the sample region of interest 76 within the sample region of interest boundary 78. Once a user has defined the sample region of interest boundary 78, a user may place one or more samples 74 in the sample region of interest 76 and within the sample region of interest boundary 78. The one or more samples 74 may be placed directly onto the object support 20 or on the sample holder 72, which is then placed on object support 20.

With reference to Figure 6, in another embodiment the user may choose to select the object tool 82 on interface 34. By selecting object tool 82, a user may draw or outline the object 22 in the one or more images 42. By drawing the object 22 on the one or more images 42, the user selects a portion of the field of view of the x-ray source 16 to exclude. The outline or drawing of the object 22 denotes which area of the field of view of the x-ray source 16 to exclude while illuminating the portions of the field of view not corresponding to the object 22. The phrase "illuminating" as used in this specification may be used within the context of illuminating an object or biopsy samples with radiation using the x-ray source 16 or may be used when referring to illumination means, where an object, the object support, and/or the biopsy samples may be illuminated by illumination means such as a light source. A person having ordinary skill in the art will understand the context of the phrase "illuminating" from whether reference is made to the x-ray source or to the illuminating means. In some embodiments, this field of view may correspond exactly to drawn object area 86 to exclude the drawn object from the x-ray field of view or the field of view may substantially correspond to the drawn object area 86 of the outlined objected based on the limitations of the collimators.

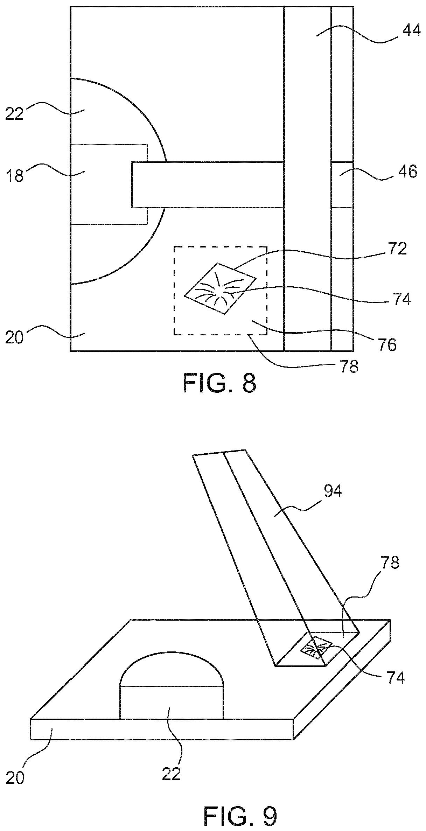

Referring to Figure 9, the samples 74 being imaged is depicted. Once a field of view has been proposed by the system 10 or drawn or selected by the user as shown in Figure 3 and Figure 6, the coordinates corresponding to said field of view 94 may then be sent from the controller 29 to a collimator 92. Based upon the coordinates, the collimator 92 makes adjustments to limit the field of view 94 to the selected region of interest. Alternatively, in any of the embodiments discussed herein, a user may program the coordinates of the field of view 94 into the collimator 92. The adjustment of the collimator to a field of view that is smaller than that which is used to acquire the one or more images 42 prevents unnecessary radiation exposure to the patient or object, and also allows the samples 74 to be imaged apart from the patient or object 22.

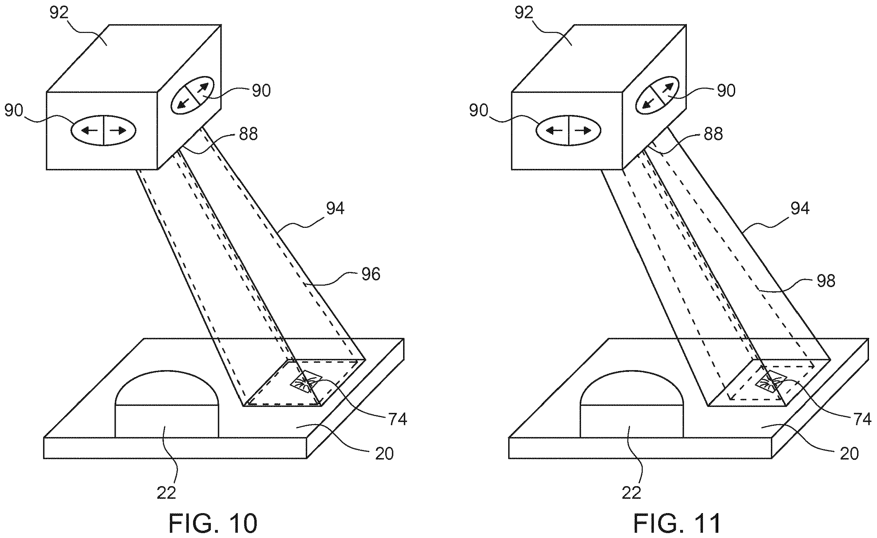

Referring to Figure 10, an embodiment of the x-ray system 10 is shown with adjustment means 90 and illuminating means 88, which may comprise a light source, for example, a light bulb. In some embodiments of the x-ray system 10, the collimator 92 is included. The collimator 92 may further include adjustment means 90, which allows the position of the field of view 94 to be moved in any direction on the object support 20. Adjustment means 90 may also be used to adjust the field of view 94 to be smaller or larger, similar to the optical adjustment on a camera.

In some embodiments of the x-ray system 10, illuminating means 88 may also be included within the collimator 92. The illuminating means 88 can be used to verify that the object 22 is outside of the field of view 94 as much as possible and/or to check that the field of view 94 is large enough to accommodate the samples 74. As illustrated in Figure 10 and Figure 11, the field of view 94 of the collimator 92 and the unadjusted field of view 96 of the illuminating means may be adjusted to accommodate the samples 74. If the user determines the field of view should be adjusted to enlarge, shrink, or move the field of view, the user may use adjustment means (not shown) to adjust the illuminating means 88, which correspond to an adjusted field of view 98 compared to the field of view 96. Alternatively, a user may use the interface 34 to adjust the field of view or may manually adjust the field of view by adjusting the collimator 92 itself.

The unadjusted field of view and the adjusted field of view may be visualized by a user using the illuminating means 88 to shine a light corresponding to the unadjusted and adjusted field of view on the object support 20. The collimator may be adjusted to correspond to the field of view of the illuminating means either directly with the adjustment of the illuminating means or after adjustment of the illuminating means. Alternatively, the unadjusted field of view and the adjusted field of view may correspond only to the collimator and the user may not be able to visually see the adjusted and unadjusted field of view. This case may occur, for example, when a user depends on the region of interest defined on the display and the collimator adjusts accordingly without any confirmation of the location of the field of view using the illuminating means.

In an embodiment depicted in Figure 10 and Figure 11, the user places the biopsy samples, either directly or indirectly, onto object support 20 prior to adjusting the field of view using the adjustment means 90 and/or using illuminating means 88 and adjustment means. In another embodiment, the field of view may be adjusted manually using the adjustment means 90 and/or using illuminating means 88 and adjustment means prior to placing the samples onto the object support 20. In yet another embodiment, the field of view may be adjusted manually using the adjustment means 90 and/or using illuminating means 88 both prior to placing the samples onto the object support 20 and after the samples are placed onto the object support 20. Once the samples have been positioned on the object support 20 and the field of view is satisfactory, at least one image of the samples is acquired. Because the patient or object has not been moved from the support, additional images may be acquired of the object and/or additional biopsy samples may be acquired. The additional images may be used to help guide the biopsy procedure as a biopsy device is inserted into the object. Additional images of the biopsy samples may also be acquired throughout the procedure.

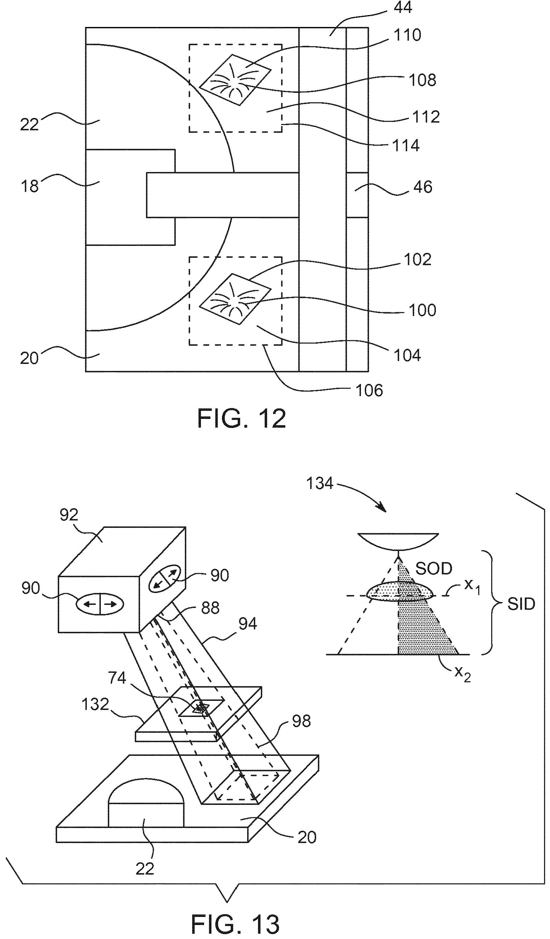

As illustrated in Figure 12, the biopsy samples may be imaged in more than one position on object support 20. In certain circumstances, such as when the object 22 is large or when the samples do not fit within a field of view that excludes a significant portion of the object 22, a user may choose to place biopsy samples in more than one position on object support 20. As shown first samples 100 on first sample holder 102 may be placed within a first sample region of interest 104 within a first sample region of interest boundary 106. Second samples 108 may be placed on second sample holder 110 within a second sample region of interest 112 within a second sample region of interest boundary 114.

In another embodiment, the x-ray system 10 automatically moves the collimator 92 to define a field of view for imaging the samples without the need of any interaction with interface 34. Of course it should be understood that a user may choose to interact with interface 34 to adjust the collimator 92 after the system 10 automatically moves the collimator 92 to define a field of view or the user may manually adjust the collimator 92. This field of view may be a field of view from a predefined set of regions of interests, may be based on knowledge of the current position of the object 22 being imaged on the object support 20, and/or may be derived from analysis of the image acquired of the object 22. Advantageously, the field of view the system 10 automatically moves the collimator 82 to would keep the object 22 outside of the field of view as much as possible and/or be a large enough field of view to accommodate the samples for sample imaging.

A user can then use the illumination means 88 to verify that the object 22 is outside of the field of view of the x-ray source 16 as much as possible and/or to check that the field of view is large enough to accommodate the samples. While guided by the illumination means 88, which may be a light, a user may determine that the field of view should be enlarged, shrunk or moved. The user can then adjust the collimation on the x-ray apparatus 12 to keep as much of the object outside of the field of view as possible, and to ensure the field of view accommodates the samples for imaging. Once the field of view is satisfactory, the user places the samples on the object support 20 inside the field of view and acquires at least one image of the samples. Alternatively, the samples may be placed on the object support 20, the x-ray system may automatically move the collimator 92 to a position, then the user may use the illumination means 88 and/or the adjustment means 90 to the samples on the object support 20.

In another embodiment, a user can use illumination means 88 corresponding to a field of view restricted by the collimator to propose and/or determine an adequate field of view. For example, after an image is acquired, a user may turn on the illumination means 88 to determine if the field of view is large enough to accommodate the samples and keep as much of the object outside of the field of view as possible. Upon using the illumination means 88, the collimator adjusts in real time along with the adjustment of the light field, or may adjust after the illumination means 88 is used, to fit the illuminated area defined by the user. The user may then place the samples on the object support 20 within the field of view and acquire at least one image of the samples. Alternatively, a user may place the samples on the object support 20 prior to turning on the illumination means 88 or prior to making any adjustments to the illumination means 88 or the collimator 92. The user may then adjust the illumination means 88 and/or collimator 92 to the sample located on the object support, so that the light field and thus the field of view excludes as much of the object being imaged as possible.

In additional embodiments, a dedicated automatic acquisition mode may be used to image the samples, for example, using pre-selected regions and techniques. The pre-selected techniques may be stored in the memory of the controller 29 and selected by a user or may be automatically selected based upon pre-defined settings, the current position of the object 22 to be imaged and/or by image analysis.

Referring to Figure 13, a user may also position the biopsy samples 74 on a support 132 mounted between the object support 20 and the x-ray source. In an embodiment, the support 132 for biopsy samples 74 mounted between the object support 20 and the x-ray source allows for the geometric magnification 134 of the biopsy samples in the acquired 2D projection image where x₂ corresponds to the plane of the object support 20, x₁ corresponds to the plane of the support 132, "SID" corresponds to the Source to Image Distance and "SOD" corresponds to the Source to Object Distance. The location of the support 132 between the object support 20 and the x-ray source allows the biopsy samples 74 to be placed closer to the x-ray source and further from detector, which results in magnification of the biopsy samples 74. In another embodiment, the support 132 mounted between the object support 20 and the x-ray source 88 may be a magnification stand.

In other embodiments, the images acquired of the samples are saved in DICOM (Digital Imaging and Communications in Medicine) format. Preferably, the DICOM images will contain the information specific to breast specimen imaging following, for example, the DICOM Supplement 122, "Specimen Module and Revised Pathology SOP Classes." This information may be manually and/or automatically filled. This labeling allows for tracking and accurate identification of the images of the biopsy sample(s).

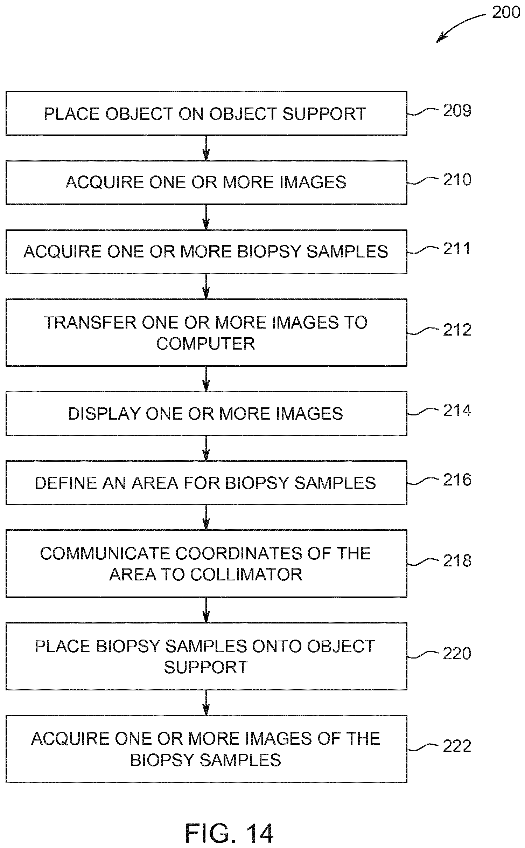

Referring to Figure 14, a flow chart 200 depicting in detail an embodiment of a method of imaging biopsy samples is shown. Prior to imaging biopsy samples, a patient may be positioned in an x-ray imaging system 10, either standing, sitting, or lying down. At step 209, the object 22 is positioned on the object support 20. The object 22 may be a patient's breast. Once the patient or object 22 is positioned on an object support 20, a compression paddle 24, is applied to the part of the patient to be scanned or the object to be scanned. In one embodiment, the compression paddle 24 has at least one aperture through which a biopsy tool may pass through to obtain biopsy samples 74.

At step 210, the x-ray apparatus 12 is used to acquire one or more images 42 of object 22. In an embodiment, the one or more x-ray images 42 comprise a scout image prior to removing biopsy samples. At step 211, one or more tissue or biopsy samples 74 are acquired from the object. In another embodiment the one or more images 42 comprise an image acquired after one or more tissue or biopsy samples 74 have been removed from the object 22. At step 212, the one or more images 42 is transferred through connection 30 to the controller 29 and may be displayed on the display screen 32 having a user interface 34 at step 214.

After the one or more images 42 is displayed, at step 216, a user may define a region of interest area 84 which corresponds to a field of view on the user interface 34 in which biopsy samples 74 are or will be placed. The area 84 corresponds to the sample region of interest 76 having region of interest boundary 78 which will be imaged by x-ray apparatus 12. To define the area 84, a user may use a mouse, keyboard, touch screen, touch pad, or other means which would allow a user to define the boundary of a region of interest for imaging biopsy samples 74, in which the biopsy samples 74 could be placed. The user would typically define the area 84 to exclude as much of the object 22 as possible while defining the area 84 to be large enough to accommodate the biopsy samples, thus limiting the amount of radiation the object 22 is exposed to.

At step 218, where the coordinates of the drawn object area 86 defined by the user are communicated to the collimator 92 as discussed above. The collimator automatically adjusts after receiving the coordinates from controller 29 or the user, or may adjust after the user has confirmed the coordinates and/or after the biopsy samples 74 have been placed onto the object support 20. At step 220, the user places one or more biopsy samples 74 directly or indirectly onto object support 20 in the area which will be illuminated by the x-ray source 16 and not in the drawn object area 86 marked by the user as the object 22, or as much outside of the drawn object area 86 as possible. At step 222, one or more images of the biopsy samples 74 is acquired.

In another embodiment of the method, the user may draw the object on the user interface 34 using an object selection or object defining tool with a mouse, keyboard, touch screen, touch pad, or other means which would allow a user to define the boundary of object 22 in the one or more images 42. In this embodiment, the user would draw or select object 22 to distinguish between the area of object support 20 that will be exposed to radiation and the area of object support 20 that will not be exposed to radiation or will be exposed to limited radiation.

The embodiment of the method then proceeds to step 218, where the coordinates of the drawn object area 86 defined by the user are communicated to the collimator 92 as discussed herein. The collimator automatically adjusts after receiving the coordinates from controller 29 or the user, or may adjust the collimator after the user has confirmed the coordinates and/or after the biopsy samples 74 have been placed onto the object support 20. At step 220, the user places one or more biopsy samples 74 directly or indirectly onto object support 20 in the area which will be illuminated by the x-ray source 16 and not in the drawn object area 86 marked by the user as the object 22, or as much outside of the drawn object area 86 as possible. At step 222, one or more images of the biopsy samples 74 is acquired.

Prior to acquiring one or more images of the biopsy samples 74 at step 222, a user may use the illuminating means 88 to aid the user in placing the biopsy samples 74 into the correct area on object support 20. The user may use the illuminating means 88 prior to placing the biopsy samples 74 onto the object support 20 so that the user would be able to visually see where to place the biopsy samples 74. The user may also use illuminating means 88 after the biopsy samples 74 have been placed onto the object support 20 to confirm the biopsy samples 74 have been placed onto the object support 20.

The user may also use illuminating means 88 to confirm object 22 is excluded entirely or as much as possible from the field of view of the x-ray source 16. In an alternative embodiment, the illuminating means 88 may illuminate the area which is excluded from the x-ray source 16 field of view rather than the area which is included within the x-ray source 16 field of view.

In an embodiment, for example, after step 218 or step 220, the user may adjust the illuminating means 88 to be larger, smaller, or of a different shape. The field illuminated by the illuminating means 88 typically corresponds to the field of view of the of x-ray source 16. Therefore, when the collimator 92 is adjusted either by the controller 29, manually by the user, or by a default setting, the field of view of the x-ray source 16 is adjusted along with the field of the illuminating means 88.

In an alternative embodiment, the collimator 92 can operate entirely or partially separate from the illuminating means 88. The user may choose to adjust the illuminating means 88 and the collimator 92 would not adjust until the user confirms the user wishes to adjust the collimator 92 to the area illuminated by the illuminating means 88. This embodiment would allow a user to make adjustments to the illuminating means to fit the biopsy samples 74 or to exclude the object 22 without moving the collimator 92 field of view until the illuminated area reaches the size and shape the user desires.

In yet another embodiment of the method depicted in Figure 14, at step 216 the area defined in which biopsy samples 74 will be placed may be comprised of selecting a predefined area, such as a predefined quadrant shown on a user interface. As shown in Figure 3, this may be done by selecting a quadrant selecting tool or selecting a quadrant directly on the one or more images 42. Alternatively, the controller 29 may propose a quadrant to the user in which to place the samples based upon a default setting or upon image analysis where the object has been identified.

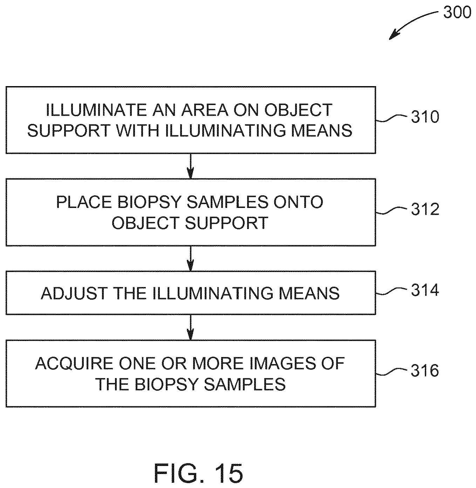

Referring to Figure 15, a flow chart 300 depicting in detail an embodiment of a method of imaging the biopsy samples 74 is shown. In method 300, at step 310 a user uses the illuminating means 88 to illuminate an area corresponding to the field of view of x-ray source 16 on or above the object support 20. Step 310 may occur before or after step 312 where biopsy samples 74 have been placed directly or indirectly onto the object support 20. In an embodiment, the user places the biopsy samples 74 onto the object support 20 after turning on illuminating means 88, but before the illuminating means 88 are adjusted. Optionally, the user adjusts the illuminating means 88 at step 314 to adjust the illuminated area to fit the biopsy samples 74 therein and/or to exclude the object 22 from the illuminated area. At step 316, one or more images of the biopsy samples is acquired.

In another embodiment, the x-ray system 10, specifically the x-ray apparatus 12, and more specifically the x-ray source 16, automatically moves to a position to illuminate an area using the collimator 92 and/or the illuminating means 88 prior to any adjustment by the user. The position the x-ray source 16 automatically moves to may be a default position, for example a position most often separate from the object 22 where biopsy samples 74 are typically placed or may automatically move to a position based off of image analysis or based off of a known position of the object 22. Once the x-ray source 16, the collimator 92, and/or the illuminating means 88 has moved to a position automatically, the user may choose to adjust one or more of the x-ray source 16, the collimator 92, and/or the illuminating means 88 to better fit the area in which the samples 74 will or have been placed and/or to exclude object 22 from the field of view of the x-ray source 16.

It should be understood that the biopsy samples 74 may be acquired at any time during the biopsy procedure or method described herein. Additional biopsy samples may be acquired after the biopsy samples have been imaged initially and throughout the biopsy procedure. For example, biopsy samples may be acquired from an object, the biopsy samples may be imaged, and the user may determine that additional biopsy samples should be taken from the object. A second, third, fourth, or further round of acquiring and imaging the biopsy samples as described herein may be performed and are within the scope of this disclosure.

Additional images may be acquired during the procedure of the object in addition to or separate from the biopsy samples. For example, a user may choose to guide a biopsy tool within a breast by inserting the tool into the patient or object and acquiring an image, moving the tool, then acquiring one or more additional images. At various times the user may also choose to image biopsy samples apart from the image. The user may then choose to guide the biopsy tool further by acquiring images of the object then, again, switch back to acquiring images of the biopsy samples.

The user may also choose to image both the biopsy samples as well as the object simultaneously during a biopsy procedure. For example, if a user wishes to confirm the position of the biopsy tool or confirm that a complete biopsy was performed of a tumor or lesion while also confirming that the biopsy samples contain the tumor or lesion the user has removed, the user may find it advantageous to image the biopsy samples and the object at the same time. The user may use the method and system described herein to adjust the areas or field of view the x-ray apparatus images.

While illustrative embodiments have been described, it is noted that various modifications will be apparent to those of ordinary skill in the art in view of the above description and drawings. For the method described herein, it is to be understood that the steps may be performed in any order in alternative embodiments and may be repeated. Such modifications are within the scope of the disclosure, which is limited and defined only by the claims.

## Claims

1. An x-ray system (10) for imaging biopsy samples (74) comprising:
an x-ray apparatus (12) comprising an x-ray source (16) including a collimator (92), an x-ray detector (18), and an object support (20), wherein the collimator (92) is adjustable to limit x-rays from the x-ray source (16) to an area of the object support (20) in which the biopsy samples (74) are placed and to exclude at least a portion of the object support (20) from the x-rays from the x-ray source (16); and
a controller (29) coupled to the x-ray apparatus (12) and a display (32) including a user interface (34), wherein the controller (29) is configured to adjust the collimator (92) to an area defined on the user interface (34) in which the biopsy samples (74) are placed;
wherein the controller (29) is further operable to:
acquire (210) one or more images (42) of a breast (22) using the x-ray system (10);
display (214) the one or more images (42) of the breast (22) on the display (32);
define (216) the area (84) on the one or more images (42) on the display (32);
communicate (218) the coordinates of the defined area (84) to the collimator (92), wherein the collimator (92) is adjustable to illuminate only the defined area (84) corresponding to a field of view; and
acquire (222) one or more images of the biopsy samples (74).

2. The x-ray system (10) of claim 1, wherein the controller (28) is operable to automatically define the area (84) on the one or more images (42).

3. The x-ray system (10) of any preceding claim, wherein the area (84) on the one or more images (42) is adjustable by a user of the x-ray system (10).

4. The x-ray system (10) of any preceding claim, configured such that the one or more images of the biopsy samples (74) are acquired without removing the breast (22) from the object support (20).

5. The x-ray system (10) of any preceding claim, further comprising a light source (88) for illuminating the defined area (84).

6. The x-ray system (10) of claim 5, further comprising an adjustment means (90) for adjusting an illuminated defined area to exclude at least a portion of the object (22).

7. The x-ray system (10) of any preceding claim, wherein the x-ray system (10) is configured to illuminate a pre-defined area of the object support (20).

## Patentansprüche

1. Ein Röntgensystem (10) zur Bildgebung von Biopsieproben (74), umfassend:
ein Röntgengerät (12), umfassend eine Röntgenquelle (16) mit einem Kollimator (92), einen Röntgendetektor (18) und einen Objektträger (20), wobei der Kollimator (92) verstellbar ist
zum Begrenzen von Röntgenstrahlen von der Röntgenquelle (16) auf einen Bereich des Objektträgers (20), in dem die Biopsieproben (74) platziert sind, und zum Ausschließen mindestens eines Teils des Objektträgers (20) von den Röntgenstrahlen von der Röntgenquelle (16); und
einen Controller (29), der an das Röntgengerät (12) und ein Display (32) mit einer Benutzerschnittstelle (34) gekoppelt ist, wobei der Controller (29) dafür ausgebildet ist, den Kollimator (92) auf einen Bereich zu verstellen, der auf der Benutzerschnittstelle (34) definiert ist, in dem die Biopsieproben (74) platziert sind;
wobei der Controller (29) weiterhin betätigt werden kann zum:
Erfassen (210) eines oder mehrerer Bilder (42) einer Brust (22) unter Verwendung des Röntgensystems (10);
Anzeigen (214) des einen oder der mehreren Bilder (42) der Brust (22) auf dem Display (32);
Definieren (216) des Bereichs (84) auf dem einen oder den mehreren Bildern (42) auf dem Display (32);
Kommunizieren (218) der Koordinaten des definierten Bereichs (84) an den Kollimator (92), wobei der Kollimator (92) verstellt werden kann
zum Beleuchten nur des definierten Bereichs (84) entsprechend einem Sichtfeld; und
Erfassen (222) eines oder mehrerer Bilder der Biopsieproben (74).

2. Röntgensystem (10) nach Anspruch 1, wobei der Controller (28) betätigt werden kann zum automatischen Definieren des Bereichs (84) auf dem einen oder den mehreren Bildern (42).

3. Röntgensystem (10) nach einem vorhergehenden Anspruch, wobei der Bereich (84) auf dem einen oder den mehreren Bildern (42) durch einen Benutzer des Röntgensystems (10) verstellt werden kann.

4. Röntgensystem (10) nach einem vorhergehenden Anspruch, das derart ausgebildet ist, dass das eine oder die mehreren Bilder der Biopsieproben (74) erfasst werden, ohne die Brust (22) von dem Objektträger (20) zu entfernen.

5. Röntgensystem (10) nach einem vorhergehenden Anspruch, weiterhin umfassend eine Lichtquelle (88) zum Beleuchten des definierten Bereichs (84).

6. Röntgensystem (10) nach Anspruch 5, weiterhin umfassend ein Verstellmittel (90) zum Verstellen eines beleuchteten definierten Bereichs, um mindestens einen Teil des Objekts (22) auszuschließen.

7. Röntgensystem (10) nach einem vorhergehenden Anspruch, wobei das Röntgensystem (10) dafür ausgebildet ist, einen vordefinierten Bereich des Objektträgers (20) zu beleuchten.

## Revendications

1. Système à rayons X (10) pour l'imagerie d'échantillons de biopsie (74) comprenant :
un appareil à rayons X (12) comprenant une source de rayons X (16) comportant un collimateur (92), un détecteur de rayons X (18) et un support d'objet (20), le collimateur (92) étant réglable pour limiter les rayons X provenant de la source de rayons X (16) sur une zone du support d'objet (20) dans laquelle sont placés les échantillons de biopsie (74) et pour exclure au moins une partie du support d'objet (20) des rayons X provenant de la source de rayons X (16) ; et
un contrôleur (29) couplé à l'appareil à rayons X (12) et un écran (32) comportant une interface utilisateur (34), le contrôleur (29) étant configuré pour régler le collimateur (92) sur une zone définie sur l'interface utilisateur (34) dans laquelle sont placés les échantillons de biopsie (74) ;
dans lequel le contrôleur (29) est également utilisable pour :
acquérir (210) une ou plusieurs images (42) d'un sein (22) en utilisant le système à rayons X (10) ;
afficher (214) la ou les images (42) du sein (22) sur l'écran (32) ;
définir (216) la zone (84) sur la ou les images (42) sur l'écran (32) ;
communiquer (218) les coordonnées de la zone définie (84) au collimateur (92), le collimateur (92) étant réglable pour éclairer uniquement la zone définie (84) correspondant à un champ de vision ; et
acquérir (222) une ou plusieurs images des échantillons de biopsie (74).

2. Système à rayons X (10) de la revendication 1, dans lequel le contrôleur (28) est utilisable pour définir automatiquement la zone (84) sur la ou les images (42).

3. Système à rayons X (10) d'une quelconque revendication précédente, dans lequel la zone (84) sur la ou les images (42) est réglable par un utilisateur du système à rayons X (10).

4. Système à rayons X (10) d'une quelconque revendication précédente, configuré de telle sorte que la ou les images des échantillons de biopsie (74) sont acquises sans retirer le sein (22) du support d'objet (20) .

5. Système à rayons X (10) d'une quelconque revendication précédente, comprenant en outre une source de lumière (88) pour éclairer la zone définie (84) .

6. Système à rayons X (10) de la revendication 5, comprenant en outre un moyen de réglage (90) pour régler une zone éclairée définie pour exclure au moins une partie de l'objet (22).

7. Système à rayons X (10) d'une quelconque revendication précédente, le système à rayons X (10) étant configuré pour éclairer une zone prédéfinie du support d'objet (20).
